# EUROPÄISCHE PATENTANMELDUNG

(11) **EP 2 314 374 A1**
(43) Veröffentlichungstag der Anmeldung: **27.04.2011**
(21) Anmeldenummer: 10180596.8
(22) Anmeldetag: 04.02.2003
(51) Int. Cl.: B01J 23/89, B01J 27/128, B01J 27/138, C07C 17/156, C07C 19/045, B01J 27/051

(54) **Katalysatorzusammensetzung zur Oxichlorierung**

(30) Priorität: 05.02.2002 DE 10204608; 07.08.2002 DE 10236254
(62) Teilanmeldung aus: 03704531.7
(71) Anmelder: BASF Corporation, Florham Park, NJ 07932 (US)
(72) Erfinder: Kuhrs, Christian, 69115 Heidelberg (DE); Meissner, Ruprecht, 67273 Weisenheim (DE)
(74) Vertreter: Altmann, Andreas

(57) **Zusammenfassung**

Die vorliegende Erfindung betrifft eine Katalysator-Zusammensetzung zur Oxychlorierung von Ethylen, enthaltend ein Gemisch aus Metallsalzen auf einem Träger, wobei die besagten Metallsalze in solchen Verhältnisanteilen auf den Träger aufgebracht werden, dass die Katalysator-Zusammensetzung
a) 3 bis 12 Gew.-% Kupfer als Kupfersalz,
b) 0 bis 3 Gew.-% eines Erdalkalimetalls als Erdalkalimetallsalz,
c) 0 bis 3 Gew.-% eines Alkalimetalls als Alkalimetallsalz,
d) 0,001 bis 0,1 Gew.-%, vorzugsweise 0,005 bis 0,05 Gew.-%, mindestens eines Metalls ausgewählt aus der Gruppe Ruthenium, Rhodium, Palladium, Osmium, Iridium und Platin und/oder 0,0001 bis 0,1 Gew.-%, vorzugsweise 0,001 bis 0,05 Gew.-% und Gold, als entsprechendes Metallsalz oder Tetrachlorogoldsäure,

umfasst, wobei alle Gewichtsprozente auf das Gesamtgewicht des Katalysators einschließlich Trägermaterial bezogen sind.

Die Erfindung betrifft weiterhin ein Verfahren zur Herstellung von 1,2 Dichlorethan zur Oxychlorierung von Ethylen unter Katalyse der obigen Katalysator-Zusammensetzung.

## Beschreibung

Die vorliegende Erfindung betrifft eine Katalysator-Zusammensetzung zur Oxychlorierung von Ethylen, enthaltend ein Gemisch aus Metallsalzen auf einem Träger, wobei die besagten Metallsalze in solchen Verhältnisanteilen auf den Träger aufgebracht werden, dass die Katalysator-Zusammensetzung 3 bis 12 Gew.-% Kupfer als Kupfersalz, 0 bis 3 Gew.-% eines Erdalkalimetalls als Erdalkalimetallsalz, 0 bis 3 Gew.-% eines Alkalimetalls als Alkalimetallsalz, 0,001 bis 0,1 Gew.-%, vorzugsweise 0,005 bis 0,05 Gew.-%, mindestens eines Metalls ausgewählt aus der Gruppe Ruthenium, Rhodium, Palladium, Osmium, Iridium und Platin und/oder 0,0001 bis 0,1 Gew.-%, vorzugsweise 0,001 bis 0,05 Gew.-% Gold, als entsprechendes Metallsalz, im Falle des Goldes auch als Tetrachlorogoldsäure (HAuCl₄), umfasst, wobei alle Gewichtsprozente auf das Gesamtgewicht des Katalysators einschließlich Trägermaterial bezogen sind.

Die Erfindung betrifft weiterhin ein Verfahren zur Herstellung von 1,2 Dichlorethan durch Oxychlorierung von Ethylen, gekennzeichnet durch die Umsetzung einer Mischung aus Ethylen, Sauerstoff oder sauerstoffenthaltendem Gas und Chlorwasserstoff zu 1,2 Dichlorethan mittels eines Katalysators der vorstehenden Zusammensetzung.

Die Oxychlorierung von Ethylen zu 1,2-Dichlorethan ist ein allgemein bekanntes Verfahren, bei dem Ethylen mit Chlorwasserstoff und Sauerstoff oder mit einem sauerstoffhaltigen Gas (z.B. Luft) in der Gasphase und üblicherweise in Gegenwart eines Katalysators umgesetzt wird. Geeignete Katalysatoren enthalten in der Regel als katalytisch aktive Komponente eine Kupferverbindung, die auf einer Trägersubstanz abgeschieden worden ist, vorzugsweise Kupferchlorid auf einer Trägersubstanz.

Es haben sich insbesondere zwei Verfahren in der Technik durchgesetzt, bei denen man den Katalysator als Festbett anordnet oder die Reaktion im Fließbett betreibt.

Es ist weiterhin bekannt, dass die alleinige Gegenwart von Kupferchlorid in einer Katalysator-Zusammensetzung für die Oxychlorierung von Nachteil ist, da Kupferchlorid bei den gebräuchlichen Reaktionstemperaturen der Oxychlorierung überaus flüchtig ist, was im Laufe der Zeit zu einem Verlust an Katalysatorwirksamkeit führt. Die eingesetzten Träger-Katalysatoren enthalten daher üblicherweise sogenannte Promotoren, durch die die Wirksamkeit von katalytisch aktivem Kupferchlorid auf Trägersubstanzen verbessert wird. Zu diesen Promotoren zählen Alkalimetallchloride, insbesondere Kaliumchlorid und Cäsiumchlorid, Erdalkalimetallchloride, insbesondere Magnesiumchlorid, oder auch Chloride der Seltenerdmetalle, insbesondere Cerchlorid. Es ist beispielsweise seit langem bekannt, dass Kupferchlorid weniger flüchtig ist, wenn man es gemeinsam mit Kaliumchlorid oder Natriumchlorid einsetzt.

So wird in EP-A 0 582 165 eine Katalysator-Zusammensetzung, umfassend einen Träger, der eine aktive Metallzusammensetzung aufweist, beschrieben, die 2 bis 8 Gew.-% Kupfer als Kupferchlorid, 0,2 bis 2 Gew.-% eines Alkalimetalls, 0,1 bis 9 Gew.-% eines Seltenerdmetalls und 0,05 bis 4 Gew.-% eines Metalls der Gruppe IIA des Periodensystems (Erdalkalimetalle) umfasst.

In EP-A 0 375 202 wird eine Katalysator-Zusammensetzung für die Oxychlorierung, umfassend ein Gemisch von Metallchloriden auf einem Träger, offenbart, die im wesentlichen ein Gemisch aus Kupferchlorid, Magnesiumchlorid und Kaliumchlorid in solchen Verhältnissen enthält, dass die Katalysator-Zusammensetzung 3 bis 9 Gew.-% Kupfer, 0,2 bis 3 Gew.-% Magnesium und 0,2 bis 3 Gew.-% Kalium umfasst.

Eine ähnliche katalytisch aktive Metallchlorid-Zusammensetzung auf einem Träger zur Oxychlorierung von Ethylen, bei der die Metallchloride in solchen Verhältnissen eingesetzt werden, dass die Zusammensetzung 3 bis 9 Gew.-% Kupfer, 1 bis 3 Gew.-% Magnesium und 0,01 bis 1 Gew.-% Kalium umfasst, wird in EP-A 0 657 212 offenbart.

Die US 4,446,249 beschreibt eine katalytisch aktive Zusammensetzung für einen Fließbett-Katalysator, bei der auf der Trägersubstanz γ-Al₂O₃, vor der Ablagerung des Kupferchlorids, 0,5 bis 3 Gew.-% mindestens eines Metalls aus der Gruppe Kalium, Lithium, Rubidium, Cäsium, Erdalkalimetalle oder Seltenerdmetalle oder Gemische dieser Elemente abgeschieden werden. Anschließend erfolgt dann die Ablagerung des katalytisch aktiven Kupferchlorids auf den Trägerpartikeln. Durch dieses in zwei Schritten vorgenommene Aufbringen der aktiven Metallkomponenten auf die Trägersubstanz neigen die nach diesem Verfahren hergestellten Katalysator-Partikel weniger dazu, aneinander zu haften. Diese Eigenschaft ist insbesondere beim Einsatz des Katalysators als Fließbett von Vorteil.

Die Selektivität, mit der im Rahmen der Oxychlorierungsreaktion Ethylen in das gewünschte Endprodukt 1,2-Dichlorethan - und nicht in die bei der Oxychlorierung üblichen Nebenprodukte - umgesetzt wird, ist stark abhängig von der verwendeten Katalysator-Zusammensetzung. Vor allem bei Reaktionen mit hohem Ethylenumsatz werden bei Verwendung der bisher gebräuchlichen Katalysator-Zusammensetzungen noch zu viele Nebenprodukte erhalten.

Die Partikel der gebräuchlichen Zusammensetzungen neigen beim Einsatz als Fließbettkatalysatoren zum Zusammenkleben ("sticking"), wodurch die Kontinuität der Reaktion gefährdet ist. Fließbettkatalysatoren für die Oxychlorierung von Ethylen zu 1,2-Dichlorethan müssen daher nicht nur bezüglich Aktivität und Selektivität optimiert werden, sondern auch ein "sticking"-freies Wirbelverhalten aufweisen. Dabei ist zu beobachten, dass kupferreiche Katalysatoren stärker zum Zusammenkleben neigen als kupferarme Katalysatoren.

Es ist die Aufgabe der vorliegenden Erfindung, eine Katalysator-Zusammensetzung für die Oxychlorierung von Ethylen bereitzustellen, deren Verwendung bei der Oxychlorierung im Vergleich zur Verwendung der bisher gebräuchlichen Katalysator-Zusammensetzungen die Umsatzrate für die Edukte Ethylen und Chlorwasserstoff erhöht, ohne dass die Selektivität zur Bildung von 1,2 Dichlorethan dabei verringert wird und die bei Verwendung des Katalysators als Fließbett "sticking"-frei ist.

Diese Aufgabe konnte durch eine Katalysator-Zusammensetzung zur Oxychlorierung von Ethylen gelöst werden, enthaltend ein Gemisch aus Metallsalzen auf einem Träger, wobei die besagten Metallsalze in solchen Verhältnisanteilen auf den Träger aufgebracht werden, dass die Katalysator-Zusammensetzung
a) 3 bis 12 Gew.-% Kupfer als Kupfersalz,
b) 0 bis 3 Gew.-% eines Erdalkalimetalls als Erdalkalimetallsalz,
c) 0 bis 3 Gew.-% eines Alkalimetalls als Alkalimetallsalz,
d) 0,001 bis 0,1 Gew.-%, vorzugsweise 0,005 bis 0,05 Gew.-%, mindestens eines Metalls ausgewählt aus der Gruppe Ruthenium, Rhodium, Palladium, Osmium, Iridium und Platin und/oder 0,0001 bis 0,1 Gew.-%, vorzugsweise 0,001 bis 0,05 Gew.-% Gold, als entsprechendes Metallsalz oder Tetrachlorogoldsäure,
umfasst, wobei alle Gewichtsprozente auf das Gesamtgewicht des Katalysators einschließlich Trägermaterial bezogen sind.

Die Gegenwart kleiner Mengen eines Salzes der Platinmetalle, zu denen Ruthenium, Rhodium, Palladium, Osmium, Iridium und Platin gehören, oder auch die Gegenwart kleiner Mengen entsprechender Goldverbindungen in der eingesetzten Katalysator-Zusammensetzung steigern den Umsatz der Edukte Ethylen und Chlorwasserstoff wesentlich, ohne jedoch die Selektivität zur Bildung von 1,2 Dichlorethan zu verringern. Dadurch wird letztlich eine höhere Ausbeute an 1,2 Dichlorethan erhalten. Bei Verwendung des Katalysators als Fließbett wird das Wirbelverhalten dabei durch die Zugabe geringer Mengen der oben erwähnten Metallsalze nicht beeinflusst. Hierbei werden als Metallsalze der Platinmetalle oder des Golds vorzugsweise die entsprechenden Oxyhalogenide, die Oxide oder die Halogenide der Platinmetalle oder des Golds, insbesondere die Chloride der Platinmetalle oder des Golds eingesetzt.

Die Gegenwart eines Rutheniumsalzes, insbesondere die Gegenwart von Rutheniumchlorid, in dem oben definierten Mengenverhältnis in der Katalysator-Zusammensetzung ist weiterhin bevorzugt.

Besonders bevorzugt ist die Gegenwart eines Goldsalzes, insbesondere von Goldchlorid bzw. Tetrachlorogoldsäure, in dem oben definierten Mengenverhältnis in der Katalysator-Zusammensetzung.

Bei Verwendung der bisher gebräuchlichen Katalysator-Zusammensetzungen zur Oxychlorierung treten als Nebenprodukte Ethan, Wasser, Spuren von Chlorwasserstoff, sowie chlorierte organische Nebenprodukte sowie Kohlenmonoxid und Kohlendioxid auf. CO und CO₂ stehen im Mengenverhältnis von ungefähr 1:1 zueinander. In Kreislaufreaktorverfahren werden diese Nebenprodukte der Oxychlorierung nach Entfernung des Hauptproduktes 1,2 Dichlorethan aus dem Produktgasstrom z.T. auskondensiert und z.T. üblicherweise über einen Purge-Strom aus dem Verfahren ausgeschleust. Anschließend wird das verbleibende Produktgas, das noch nicht umgesetztes Edukt, insbesondere Ethylen, enthält, in den Reaktor zurückgeführt. Bei der Entfernung von Nebenprodukten über einen Purge-Strom entstehen allerdings auch geringfügige Verluste an 1,2 Dichlorethan, dem Hauptprodukt. Die Vermeidung der Bildung von CO könnte Vorteile in der Aufarbeitung von Abgas- bzw. Kreisgasströmen bieten, da CO₂ im Unterschied zu CO leichter ausgewaschen werden kann.

Der Einsatz einer erfindungsgemäßen Katalysator-Zusammensetzung bei der Oxychlorierung bietet daher neben der Steigerung der Ausbeute an 1,2 Dichlorethan bei Verwendung eines Promotors aus der Gruppe der Platinmetalle den weiteren Vorteil, dass nur noch sehr geringe Mengen an Kohlenmonoxid entstehen, während Kohlendioxid als fast ausschließliches Nebenprodukt gebildet wird.

Als Trägersubstanz für die erfindungsgemäße Katalysator-Zusammensetzung kann Aluminiumoxid, Silicagel, Bimsstein und Ton eingesetzt werden. Bevorzugt ist der Einsatz von Aluminiumoxid als Trägersubstanz. Die spezifische Oberfläche der Trägersubstanz vor der Metallsalz-Ablagerung liegt vorzugsweise im Bereich von 20 bis 400 m²/g, bevorzugter bei 75 bis 200 m²/g. Gebräuchliche Trägersubstanzen für Oxychlorierungskatalysatoren verfügen vorzugsweise über Porenvolumen im Bereich von 0,15 bis 0,75 cm³/g, und die durchschnittlichen Teilchengrößen liegen vorzugsweise im Bereich von 30 bis 500 µm. Bei den hier verwendeten Trägersubstanzen beträgt der Anteil der Partikel mit einem Durchmesser kleiner 45 µm 30 % bzw. 5 %, wobei die BET-Oberflächen 170 m²/g bzw. 150 m²/g betragen.

Die Erfindung betrifft weiterhin ein Verfahren zur Herstellung von 1,2 Dichlorethan zur Oxychlorierung von Ethylen unter Katalyse einer erfindungsgemäßen Katalysator-Zusammensetzung.

Bei der Durchführung des erfindungsgemäßen Verfahrens zur Herstellung von 1,2 Dichlorethan können die bereits bekannten Techniken und Reaktionsbedingungen, die nach dem Stand der Technik allgemein eingeführt sind, verwendet werden. Es werden Ethylen, Chlorwasserstoff, molekularer Sauerstoff mit einer erfindungsgemäßen Katalysator-Zusammensetzung bei Temperaturen von 80 bis 300°C, bevorzugt bei 210 bis 260°C in der Gasphase in Kontakt gebracht. Der molekulare Sauerstoff kann hierbei als solcher oder in Form eines sauerstoffhaltigen Gasgemisches, z.B. Luft, eingeführt werden. Bei Kreislaufreaktorverfahren (Kreisgasfahrweise), bei denen nicht umgesetztes Edukt in den Reaktor zurückgeführt wird, wird ausschließlich reiner Sauerstoff verwendet.

Die molaren Verhältnisse der in dem erfindungsgemäßen Verfahren eingesetzten Edukte beträgt für Chlorwasserstoff:Sauerstoff im allgemeinen 5:1 bis 3:1, vorzugsweise ca. 4:1 und für Ethylen:Chlorwasserstoff im allgemeinen ca. 1:2. Vorzugsweise liegt Chlorwasserstoff, bezogen auf die Reaktion 2 C₂H₄ + 4 HCl + O₂ ^{a} 2 C₂H₄Cl₂ + 2 H₂O, in leicht unterstöchiometrischer Menge vor, so dass sichergestellt ist, dass Chlorwasserstoff in einem Reaktordurchgang nahezu vollständig umgesetzt wird. Bei der Kreisgasfahrweise wird Ethylen in noch höherem Überschuss gefahren. Das Verhältnis Ethylen:HCl:O₂ wird vorzugsweise so gewählt, dass auch Ethylen in einem Reaktordurchgang möglichst weitgehend umgesetzt wird. Der Reaktionsdruck liegt im Bereich von 1 bis 20 bar, bevorzugt bei 1 bis 8 bar.

Der verwendete Reaktorwerkstoff beruht üblicherweise auf Eisen-Basis (Edelstahl) oder auf Basis einer Nickel-Legierung. Bei Durchführung einer Oxychlorierungsreaktion im kleinen Maßstab kann auch Glas als Reaktorwerkstoff eingesetzt werden.

Der Katalysator kann im erfindungsgemäßen Verfahren sowohl als Festbett wie auch als Fließbett eingesetzt werden. Wird der Katalysator im erfindungsgemäßen Verfahren als Fließbett eingesetzt, so befindet er sich vorzugsweise im fließfähigen Zustand. Dieser tritt im allgemeinen bei Geschwindigkeiten im Bereich von 1 bis 100 cm/s ein. Wird der Katalysator im erfindungsgemäßen Verfahren als Festbett verwendet, so wird er vorzugsweise in Form von Hohlzylindern oder Ringtabletten eingesetzt, deren Stirnflächen sowohl zum Außenrand als auch zum Rand der Innenbohrungen hin abgerundet sind. Bei dieser bevorzugt eingesetzten Festbettkatalysator-Form handelt es sich entweder um aus katalytisch aktiven Material aufgebauten Hohlzylindern oder Ringtabletten, vorzugsweise um ein zu Hohlzylindern oder Ringtabletten geformtes Trägermaterial, auf das eine katalytisch aktive Masse aufgebracht ist. Der Außendurchmesser der Katalysator-Hohlzylinder bzw. -Ringtabletten beträgt 3 bis 20 mm, bevorzugt 3 bis 10 mm, besonders bevorzugt 3 bis 7 mm, insbesondere 3,5 bis 6,5 mm und der Innendurchmesser liegt bei dem 0,1- bis 0,7-fachen des Außendurchmessers. Die Länge der Katalysator-Hohlzylinder bzw. -Ringtabletten beträgt das 0,2- bis 2-fache, bevorzugt das 0,3- bis 1,8-fache, besonders bevorzugt das 0,4- bis 1,6-fache des Außendurchmessers. Der Radius der Krümmung der Stirnflächen beträgt das 0,01- bis 0,5-fache, bevorzugt das 0,05- bis 0,4-fache, besonders bevorzugt das 0,1- bis 0,2-fache des Außendurchmessers. Diese Katalysatorformen, die in EP 1 127 618 A1 beschrieben werden, zeichnen sich besonders durch einen geringen Druckverlust, sowie durch eine gute mechanische Belastbarkeit aus und eignen sich besonders gut für den Einsatz bei stark exothermen Reaktionen wie der Oxychlorierung von Ethylen.

Festbett-Katalysatoren einer Form wie in EP 1 127 618 A1 beschrieben sind damit ein integraler Bestandteil der vorliegenden Erfindung und durch Bezugnahme eingeschlossen. Die Erfindung betrifft weiterhin ein Verfahren zur Herstellung von 1,2 Dichlorethan durch Oxychlorierung von Ethylen unter Katalyse eines Festbettkatalysators mit einer erfindungsgemäßen Zusammensetzung und gegebenenfalls einer Form nach EP 1 127 618 A1.

Zur Herstellung einer wässrigen Lösung zur Imprägnierung der Trägersubstanz werden die erforderlichen Mengen der geeigneten Metallverbindungen, vorzugsweise in Form ihrer Hydrate, in Wasser gelöst. Die wässrige Lösung wird dann auf die Trägersubstanz aufgebracht. Die so imprägnierte Trägersubstanz wird anschließend gegebenenfalls von der verbleibenden wässrigen Phase abfiltriert und zum Schluss getrocknet. Das Abfiltrieren ist dann nicht notwendig, wenn die Trägersubstanz mit einem Volumenanteil der wässrigen Lösung in Kontakt gebracht wird, der nicht größer ist als zur Sättigung der Trägersubstanz ausreicht.

Die vorliegende Erfindung wird durch die nachfolgenden Beispiele veranschaulicht.

### Beispiel 1

Eine erfindungsgemäße Katalysator-Zusammensetzung wurde durch Imprägnierung der Trägersubstanz mit einer wässrigen Lösung hergestellt, die wie folgt erhalten wurde:
86,4 g CuCl*2H₂O, 89,8 g MgCl*6H₂O, 5,5 g KCl und 0,2 g RuCl₃*H₂O wurden in einer kleinen Menge Wasser gelöst. Weiteres Wasser wurde hinzugefügt, bis ein Gesamtvolumen von 300 ml - gemäß der maximalen Wasserabsorptionskapazität der eingesetzten Menge an Trägersubstanz - erhalten wurde. Diese Metallchlorid-Lösung wurde auf 600 g eines Aluminiumoxid-Trägers mit einem Partikelgrößenanteil unter 45 µm von 30 % und einer BET-Oberfläche von 170 m²/g gegeben. Nach einstündigem Rühren wurde das Gemisch bei 110°C in Gegenwart von Stickstoff 16 Stunden getrocknet, um Katalysator A mit 4,5 Gew.-% Cu, 1,5 Gew.-% Mg, 0,4 Gew.-% K und 0,01 Gew.-% Ru zu erhalten.

Zu Vergleichszwecken wurde auf gleiche Weise eine Katalysator-Zusammensetzung mit den gleichen Gewichtsanteilen an Kupferchlorid, Magnesiumchlorid und Kaliumchlorid, aber ohne Rutheniumchlorid, hergestellt (Katalysator B).

Beide Oxychlorierungskatalysatoren (Katalysator A und B) wurden in einem Fließbettreaktor aus Glas, dem die Edukte Ethylen, Luft und Chlorwasserstoff zugeführt und der bei 232°C, 243°C bzw. 254°C Bett-Temperatur gehalten wurde, verwendet. Der Reaktor wurde bei einem Druck von 4 bar mit einer Menge von 500 g des Katalysators der entsprechenden Zusammensetzung betrieben. Dem Reaktor wurden jeweils 119 Nl/h Luft, 69,9 Nl/h Chlorwasserstoff und 35,5 Nl/h Ethylen zugeführt, was eine Raumzeit von 160 g Kat./(mol HCl h-1) erbrachte. Die jeweils gebildeten Produkte wurden mit Hilfe der Gaschromatographie analysiert.

Die Ergebnisse sind in der nachfolgenden Tabelle zusammengestellt.

Ein Vergleich der Ergebnisse zeigt, daß die Anwesenheit geringer Mengen von Rutheniumchlorid in der verwendeten Katalysator-Zusammensetzung bei unterschiedlichen Reaktionstemperaturen die Umsatzrate sowohl für Chlorwasserstoff als auch für Ethylen steigert, ohne die Selektivität für 1,2 Dichlorethan zu verringern (siehe nachfolgende Tabelle). Dies wirkt sich in einer deutlichen Erhöhung der Ausbeute an 1,2 Dichlorethan bei Verwendung der erfindungsgemäßen Katalysator-Zusammensetzung (hier Katalysator A) aus.

Zusätzlich zeigen die Ergebnisse, daß die Bildung von Kohlenmonoxid als Nebenprodukt durch Verwendung der erfindungsgemäßen Katalysator-Zusammensetzung wirksam unterdrückt wird.

### Beispiel 2

Eine weitere erfindungsgemäße Katalysatorzusammensetzung wurde entsprechend Beispiel 1 hergestellt, jedoch wurde statt Rutheniumchlorid Palladiumchlorid als Promotor verwendet, um Katalysator C mit 4,5 Gew.-% Cu, 1,5 Gew.-% Mg, 0,4 Gew.-% K und 0,01 Gew.-% Pd zu erhalten.

Die Ergebnisse sind in der nachfolgenden Tabelle zusammengestellt. Deutlich wird daraus die dem Ruthenium vergleichbare Wirkung des Palladiums, sowohl bezüglich der Steigerung der Aktivität als auch bezüglich der Unterdrückung der Bildung von CO.

### Beispiel 3

Eine weitere erfindungsgemäße Katalysatorzusammensetzung wurde entsprechend Beispiel 1 hergestellt, jedoch wurde statt Rutheniumchlorid Goldchlorid als Promotor verwendet, um Katalysator D mit 4,5 Gew.-% Cu, 1,5 Gew.-% Mg, 0,4 Gew.-% K und 0,005 Gew.-% Au zu erhalten.

Die Ergebnisse sind in der nachfolgenden Tabelle zusammengestellt. Deutlich wird daraus die im Vergleich zu Ruthenium und Palladium noch stärker aktivitätssteigende Wirkung des Goldes - insbesondere bei den beiden niedrigeren Temperaturen.

**Tabelle**

| Katalysator | Temp (°C) | HCl-Umsatz (%) | Ethylen-Umsatz (%) | EDC-Selektivität (%) | EDC-Ausbeute (%) | CO-Selektivität (%) | CO₂-Selektivität (%) | Selektivität zur Bildung chlorierter Nebenprodukte (Summe, %) |
|---|---|---|---|---|---|---|---|---|
| A | 254 | 98,93 | 98,54 | 95,87 | 94,47 | 0,07 | 2,17 | 1,88 |
| A | 243 | 97,42 | 96,48 | 97,41 | 93,98 | 0,06 | 1,22 | 1,31 |
| A | 232 | 93,41 | 91,97 | 98,26 | 90,37 | 0,03 | 0,51 | 1,20 |
| B | 254 | 97,90 | 97,42 | 96,08 | 93,60 | 1,04 | 1,00 | 1,88 |
| B | 243 | 95,18 | 94,37 | 97,41 | 91,93 | 0,84 | 0,52 | 1,22 |
| B | 232 | 89,19 | 88,21 | 98,55 | 86,94 | 0,53 | 0,23 | 0,68 |
| C | 254 | 98,81 | 98,71 | 95,78 | 94,55 | 0,13 | 2,32 | 1,77 |
| C | 243 | 97,60 | 97,11 | 97,27 | 94,46 | 0,29 | 1,36 | 1,08 |
| C | 232 | 96,21 | 94,34 | 97,86 | 92,32 | 0,19 | 0,80 | 1,15 |
| D | 254 | 99,54 | 99,02 | 95,22 | 94,28 | 1,04 | 1,32 | 2,43 |
| D | 243 | 98,82 | 98,00 | 97,26 | 95,31 | 0,69 | 0,77 | 1,27 |
| D | 232 | 96,68 | 95,90 | 98,28 | 94,25 | 0,49 | 0,53 | 0,69 |

## Patentansprüche

1. Katalysator-Zusammensetzung zur Oxychlorierung von Ethylen, enthaltend ein Gemisch aus Metallsalzen auf einem Träger, wobei die besagten Metallsalze in solchen Verhältnisanteilen auf den Träger aufgebracht werden, dass die Katalysator-Zusammensetzung
a) 3 bis 12 Gew.-% Kupfer als Kupfersalz,
b) 0 bis 3 Gew.-% eines Erdalkalimetalls als Erdalkalimetallsalz,
c) 0 bis 3 Gew.-% eines Alkalimetalls als Alkalimetallsalz,
d) 0,001 bis 0,1 Gew.-%, vorzugsweise 0,005 bis 0,05 Gew.-%, mindestens eines Metalls ausgewählt aus der Gruppe Ruthenium, Rhodium, Palladium, Osmium, Iridium und Platin und/oder 0,0001 bis 0,1 Gew.-%, vorzugsweise 0,001 bis 0,05 Gew.-% Gold, als entsprechendes Metallsalz oder Tetrachlorogoldsäure,
umfasst, wobei alle Gewichtsprozente auf das Gesamtgewicht des Katalysators einschließlich Trägermaterial bezogen sind.

2. Katalysator-Zusammensetzung nach Anspruch 1, **dadurch gekennzeichnet, dass** die Metallsalze ausgewählt sind aus den Metallhalogeniden, Metalloxyhalogeniden und Metalloxiden des jeweiligen Metalls und Tetrachlorogoldsäure.

3. Katalysator-Zusammensetzung nach Anspruch 2, **dadurch gekennzeichnet, dass** die Metallhalogenide, vorzugsweise die Metallchloride des jeweiligen Metalls eingesetzt werden.

4. Katalysator-Zusammensetzung nach einem der Ansprüche 1 bis 3, **dadurch gekennzeichnet, dass** als Komponente d) ein Rutheniumsalz oder ein Goldsalz eingesetzt wird.

5. Katalysator-Zusammensetzung nach mindestens einem der Ansprüche 1 bis 4, **dadurch gekennzeichnet, dass** als Komponente b) ein Magnesiumsalz eingesetzt wird.

6. Katalysator-Zusammensetzung nach mindestens einem der Ansprüche 1 bis 5, **dadurch gekennzeichnet, dass** als Komponente c) ein Kaliumsalz eingesetzt wird.

7. Katalysator-Zusammensetzung nach einem der Ansprüche 1 bis 6, **dadurch gekennzeichnet, dass** als Träger Aluminiumoxid eingesetzt wird.

8. Katalysator-Zusammensetzung nach einem der Ansprüche 1 bis 7, **dadurch gekennzeichnet, dass** der Träger ein Porenvolumen im Bereich von 0,15 bis 0,75 cm³/g besitzt.

9. Katalysator-Zusammensetzung nach einem der Ansprüche 1 bis 8, **dadurch gekennzeichnet, dass** die spezifische Oberfläche des eingesetzten Trägers im Bereich von 20 bis 400 m²/g liegt.

10. Katalysator-Zusammensetzung nach einem der Ansprüche 1 bis 7, **dadurch gekennzeichnet, dass** sie als Festbett- Katalysator eingesetzt wird und die Form von Hohlzylindern oder Ringtabletten aufweist, deren Stirnflächen sowohl zum Außenrand als auch zum Rand der Innenbohrungen hin abgerundet sind.

11. Verfahren zur Herstellung von 1,2 Dichlorethan zur Oxychlorierung von Ethylen unter Katalyse einer Katalysator-Zusammensetzung nach einem der Ansprüche 1 bis 10.

12. Verfahren gemäß Anspruch 11, **dadurch gekennzeichnet, dass** es sich um ein Kreislaufreaktorverfahren handelt.

13. Verfahren nach mindestens einem der Ansprüche 11 oder 12, **dadurch gekennzeichnet, dass** der Katalysator als Fließbett eingesetzt wird.

14. Verfahren nach mindestens einem der Ansprüche 11 oder 12, **dadurch gekennzeichnet, dass** der Katalysator als Festbett eingesetzt wird.

15. Verfahren nach Anspruch 14, **dadurch gekennzeichnet, dass** der Katalysator als Festbett in Form von Hohlzylindern oder Ringtabletten eingesetzt wird, deren Stirnflächen sowohl zum Außenrand als auch zu den Innenbohrungen hin abgerundet sind.
